# EUROPEAN PATENT APPLICATION

(11) **EP 4 563 082 A1**
(43) Date of publication of application: **04.06.2025**
(21) Application number: 23213211.8
(22) Date of filing: 30.11.2023
(51) Int. Cl.: A61B 5/024, A61B 5/1455, A61B 5/00

(54) **NASAL SENSOR, SYSTEM AND METHOD FOR DETERMINING PHYSIOLOGICAL INFORMATION OF A SUBJECT**

(71) Applicant: Koninklijke Philips N.V., 5656 AG Eindhoven (NL)
(72) Inventor: JANSSEN, Anton, Eindhoven (NL); WIJSHOFF, Ralph Wilhelm Christianus Gemma Rosa, Eindhoven (NL); MUEHLSTEFF, Jens, 5656AG Eindhoven (NL); MENA BENITO, Maria Estrella, Eindhoven (NL)
(74) Representative: Philips Intellectual Property & Standards

(57) **Abstract**

The present invention relates to a nasal sensor (10, 110, 210), system and method for determining physiological information of a subject. The nasal sensor comprises a nasal mount (11, 12, 13), a light emitter (20, 21) secured to the first support and configured to emit light in at least one wavelength range towards the second nostril, a light detector (30, 31) secured to the first or second support and configured to detect light in the at least one wavelength range, and one or more detection modification elements (40, 41, 42, 43, 45) secured to the second support and configured to change the amount of light detected by the light detector based on the direction of flow of air within the second nostril.

## Description

### FIELD OF THE INVENTION

The present invention relates to a nasal sensor and to a system and method for determining physiological information of a subject, e.g. a patient or other person. Physiological information may particularly be information regarding the subject's heart rate, respiration rate, oxygen saturation or other vital signs.

### BACKGROUND OF THE INVENTION

Nasal sensors that measure airflow using a thermistor are known, e.g., from EP 2866655 A1. During exhalation, warm air flows over the thermistor which increases its temperature and consequently alters its resistance. By detecting changes in resistance, individual exhalations can be detected, from which respiration rate can be derived.

In current implementations of such nasal sensors, only changes in resistance can be detected, which does not provide information about the respiratory cycle, such as the onsets of inhalation. Such nasal sensors are thus insensitive to the direction of flow and the detection depends on the temperature difference between exhaled and ambient air. Both (warm) air flowing out of and into the nose will cause a change in temperature and resistance of the thermistor. Consequently, (warm) air flowing into the nose, e.g. due to presence of a fan, can cause false detections, leading to erroneous respiration rates. Moreover, only onsets of exhalation can be observed in the signal, when warm air passes the sensor, but onsets of inhalation cannot be accurately observed, when air at ambient temperature passes the sensor so that the respiration cycles cannot be determined. Furthermore, in case of shallow breathing and/or a small temperature difference between exhaled and ambient air, the change in temperature of the thermistor will be small, which complicates accurate detection of the exhalation.

### SUMMARY OF THE INVENTION

It is an object of the present invention to enable direction-sensitive detection of respiratory information and other physiological information.

In a first aspect of the present invention a nasal sensor is presented comprising:
- a nasal mount configured to be secured to nose of a subject and including a first support and a second support with the nasal septum or an alar wing interposed between the first support and the second support and with the first support arranged within a first nostril or outside the nostrils at an alar wing of the second nostril and with the second support arranged inside the second nostril;
- a light emitter secured to the first support and configured to emit light in at least one wavelength range towards the second nostril;
- a light detector secured to the first or second support and configured to detect light in the at least one wavelength range; and
- one or more detection modification elements secured to the second support and configured to change the amount of light detected by the light detector based on the direction of flow of air within the second nostril.

In a further aspect of the present invention a system for determining physiological information of a subject is presented comprising:
- a nasal sensor, in particular as disclosed herein, the nasal sensor comprising:
   a nasal mount configured to be secured to nose of a subject and including a first support and a second support with the nasal septum or an alar wing interposed between the first support and the second support and with the first support arranged within a first nostril or outside the nostrils at an alar wing of the second nostril and with the second support arranged inside the second nostril;
   a light emitter secured to the first support and configured to emit light in at least one wavelength range towards the second nostril; and
   a light detector secured to the first or second support and configured to detect light in the at least one wavelength range;
- a processing unit configured to derive a photoplethysmography, PPG, signal from the light detected by the light detector, to determine one or more vital signs from the PPG signal and to determine the direction of respiratory flow through the subject's nose, and optionally respiration rate, from the temporal variation of the amount of light detected by the light detector; and
- a signal line configured to provide a detection signal representing the light detected by the light detector to the processing unit.

In a yet further aspect of the present invention a method for determining physiological information of a subject by use of a nasal sensor, in particular as disclosed herein, is presented, the nasal sensor comprising:
a nasal mount configured to be secured to nose of a subject and including a first support and a second support with the nasal septum or an alar wing interposed between the first support and the second support and with the first support arranged within a first nostril or outside the nostrils at an alar wing of the second nostril and with the second support arranged inside the second nostril;
a light emitter secured to the first support and configured to emit light in at least one wavelength range towards the second nostril; and
a light detector secured to the first or second support and configured to detect light in the at least one wavelength range,
the method comprising:
   - deriving a photoplethysmography, PPG, signal from the light detected by the light detector;
   - determining one or more vital signs from the PPG signal; and
   - determining the direction of respiratory flow through the subject's nose, and optionally respiration rate, from the temporal variation of the amount of light detected by the light detector.

Preferred embodiments of the invention are defined in the dependent claims. It shall be understood that the claimed system and method have similar and/or identical preferred embodiments as the claimed nasal sensor, in particular as defined in the dependent claims and as disclosed herein.

The present invention is based on the idea to provide a nasal sensor, system and method that perform a direction-sensitive respiratory flow measurement and a processing of the signals from the direction-sensitive flow measurement to obtain physiological information of the subject. By using a flow measurement which is sensitive to flow direction, it can be distinguished between outflowing and inflowing air. This enables robust detection of only exhalations and allows for determination of respiration rate which is robust to disturbing external (in-going) air flows. Moreover, this allows identifying inhalation cycles which enables oxygen concentrators to provide oxygen only upon inhalation, which saves power.

The present invention allows to derive a PPG signal from light detected by the light detector of the nasal sensor, from which one or more vital signs can be determined. In parallel, the direction of respiratory flow through the subject's nose and/or respiration rate can be determined from the temporal variation of the amount of light detected by the light detector. Thus, the light detected by a single nasal sensor is used for various purposes.

The nasal sensor of the present invention comprises one or more detection modification elements that are configured to change the amount of light detected by the light detector based on the direction of flow of air within the second nostril. Thus, based on the detection of the different amounts of light, the flow direction of air can be detected. There are multiple embodiments to implement the one or more detection modification elements.

According to a preferred embodiment, the one or more detection modification elements are configured and/or located to change the amount of light detected by the light detector when air flows within the second nostril in a first direction compared to when air flows within the second nostril in a second direction substantially opposite to the first direction.

According to another embodiment, the one or more detection modification elements comprise a thermosensitive element, in particular a thermosensitive component or layer or coating, configured to change its transmissivity for light depending on its temperature. This change of transmissivity thus happens depending on the flow direction of air. If the subject inhales, colder air generally flows into the nose compared to exhalation of air through the nose.

According to an implementation of this embodiment, the light detector comprises a single light detection element and the thermosensitive element is arranged on a front side of the light detection element facing the light emitter. Thus, the thermosensitive element directly influences the amount of light that is transmitted through it from the light emitter to the light detector. The rear side of the light detection element may be covered by an opaque layer.

According to an alternative implementation of this embodiment, the light detector comprises two light detection elements, wherein a first light detection element is configured to detect light in the at least one wavelength range and a second light detection element is configured to detect light in the same and/or a different wavelength range, and
wherein the thermosensitive element is arranged within or on or adjacent to the second light detection element, in particular on a light sensitive surface side of the second light detection element. Thus, the first light detection element is particularly used for detecting light from which a PPG signal is derived, whereas the second light detection element is used for detecting the direction of airflow based on the varying amount of light transmitted through the thermosensitive element. The light sensitive surface side of the second light detection element can hereby be arranged to face the light emitter or facing away from the light emitter.

Preferably, the one or more detection modification elements further comprise a heating element configured to heat air flowing into the direction of the thermosensitive element. The heating element thus further enhances the effect of the thermosensitive element to change the amount of light transmission depending on the direction of airflow because it further heats up the air flowing from the heating element to the thermosensitive element whereas it does not heat up air flowing in the opposite direction. The heating element may be arranged between the opening of the second nostril and the thermosensitive element or, seen from the opening of the second nostril, behind the thermosensitive element. The heating element may be a separate heating element or, as particularly useful for SpO2 sensors, may be the LEDs themselves since they produce heat during operation.

In a further implementation of this embodiment, two detection elements, each with a thermosensitive element, may be provided, located on either side (in the direction of airflow) of the heating element, i.e., the heating element being arranged between the two detection elements with the respective thermosensitive element. This improves the detection of the direction of airflow and thus enables to better distinguish between exhalation and inhalation.

In another embodiment the one or more detection modification elements further comprise a contrast-enhancement element configured to enhance the thermal contrast of the thermosensitive element. This contrast-enhancement element may e.g. be a chemical layer, such as a hydrophilic layer on one side of the light detector, to enhance the thermo-contrast.

According to another embodiment, the one or more detection modification elements comprise a lid configured to change its position and/or orientation with respect to the light detector depending on the direction of flow of air within the second nostril. This represents an optical solution to change the amount of light detected by the light detector depending on direction of airflow.

The lid may be configured to move between a first position and/or orientation with respect to the light detector, when the air flows within the second nostril in a first direction, and a second position and/or orientation with respect to the light detector, when the air flows within the second nostril in a second direction substantially opposite to the first direction. In the first position and/or orientation the lid may reflect light towards the light detector whereas it does not reflect light towards the light detector in the second position and/or orientation. The lid may thus be covered with an optically reflective layer and/or made of an optically reflective material.

According to an implementation of this embodiment, the light detector comprises a single light detection element, wherein the lid is arranged on a rear side of the light detection element facing away from the light emitter, and wherein the lid is configured to move between a first position, in which the lid is arranged on or adjacent to the rear side of the light detection element, and a second position, in which the lid is arranged at a distance from or inclined to the rear side of the light detection element. This provides the desired effect of reflecting light towards the light detector in a certain position / orientation.

In a further implementation of this embodiment, two detection elements are provided, optionally arranged next to each other, each with an opto-mechanical unit (i.e., lid), where the opto-mechanical units are arranged on opposing sides of the light sensitive surfaces of the two detection elements. This improves the detection of the direction of airflow and thus enables to better distinguish between exhalation and inhalation.

According to an alternative implementation of this embodiment, the light detector comprises two light detection elements, wherein a first light detection element is configured to detect light in the at least one wavelength range and a second light detection element is configured to detect light in the same and/or a different wavelength range, and
wherein the lid is arranged on a light sensitive surface side of the second light detection element, and wherein the lid is configured to move between a first position, in which the lid is arranged on or adjacent to the light sensitive surface side of the second light detection element, and a second position, in which the lid is arranged at a distance from or inclined to the light sensitive surface side of the second light detection element. As explained above with respect to an embodiment using a thermosensitive element, the first light detection element is particularly used for detecting light from which a PPG signal is derived, whereas the second light detection element is used for detecting the direction of airflow based on the varying amount of light detected by the light detector.

The lid may be hinged or mounted with one end to the light detection element or the second light detection element, respectively, in particular to an edge of the respective light detection element, and may be configured to rotate within an angular range around its end in response to the flow of air within the second nostril. Other mechanical constructions of the lid and its arrangement with respect to the light detector are possible as well.

In another embodiment the light emitter and the light detector are secured to the first support configured to be arranged outside the nostrils at an alar wing of the second nostril, and the one or more detection modification elements are configured to reflect light emitted by the light emitter to the light detector and to change the reflectivity based on the direction of flow of air within the second nostril. The detection modification elements are thus arranged inside the second nostril and change the amount of detected light depending on the temperature of the air flowing through the second nostril, which in turn is dependent on the direction of the air flow. For instance, a thermosensitive element like a thermofoil arranged on the surface of the second support facing the first support may be used as detection modification elements. Through this change of the reflectivity, the amount of light detected by the light detector is changed so that the direction of air flow can be determined with such an embodiment as well.

In another embodiment the light emitter is configured to emit light in at least two or more different wavelength ranges towards the second nostril and the light detector is configured to detect light in the at least two wavelength ranges. For instance, red and infrared light may be used, which is particularly useful if SpO2 shall be detected from the detected light in these two wavelength ranges using the well-known ratio-of-ratios method.

The nasal sensor may further comprise a processing unit secured to the first support or the second support and configured to derive a photoplethysmography, PPG, signal from the light detected by the light detector and to determine a vital sign, in particular pulse rate and/or SpO2 and/or respiration rate, from the PPG signal and the direction of respiratory flow through the subject's nose, and optionally respiration rate, from the temporal variation of the amount of light detected by the light detector. Thus, various kinds of physiological information can be acquired by use of the nasal sensor. If respiration rate is determined from both the PPG signal and the direction of respiratory flow, both measurements of respiration rate can be combined to obtain a more robust measurement of respiration rate.

Alternatively, the processing unit is not part of the nasal sensor, but an external element, e.g. part of a portable or stationary patient monitor or other external device that processes and optionally outputs (e.g. displays) the determined information (e.g. vital signs, respiratory flow, respiration rate, etc.).

Generally, the acquired information can also be used as input to another device, e.g. to control an oxygen concentrator to provide oxygen only during inhalations (to save power) or to control a CPAP (continuous positive airway pressure) device.

### BRIEF DESCRIPTION OF THE DRAWINGS

These and other aspects of the invention will be apparent from and elucidated with reference to the embodiment(s) described hereinafter. In the following drawings
Fig. 1 shows the arrangement of an embodiment of a nasal sensor according to the present invention mounted at a subject's nose.
Fig. 2 shows a schematic diagram of the embodiment of the nasal sensor shown in Fig. 1.
Fig. 3 shows a perspective view of the embodiment of the nasal sensor shown in Fig. 1 in open state.
Fig. 4 shows a perspective view of the embodiment of the nasal sensor shown in Fig. 1 in closed state.
Fig. 5 shows a schematic diagram of a first embodiment of a nasal sensor according to the present invention.
Fig. 6 shows a schematic diagram of a second embodiment of a nasal sensor according to the present invention.
Fig. 7 shows a schematic diagram of a third embodiment of a nasal sensor according to the present invention.
Fig. 8 shows a schematic diagram of a fourth embodiment of a nasal sensor according to the present invention.
Fig. 9 shows a schematic diagram of a fifth embodiment of a nasal sensor according to the present invention.
Fig. 10 shows a schematic diagram of a sixth embodiment of a nasal sensor according to the present invention.
Fig. 11 shows a schematic diagram an embodiment of a system according to the present invention.
Fig. 12 shows a flowchart of an embodiment of a method according to the present invention.
Fig. 13 shows a schematic diagram of a seventh embodiment of a nasal sensor according to the present invention.
Fig. 14 shows a schematic diagram of an eighth embodiment of a nasal sensor according to the present invention.

### DETAILED DESCRIPTION OF EMBODIMENTS

Photoplethysmography (PPG) and pulse oximeter devices measure photoplethysmography (PPG) signals by light transmission through the nasal septum or an ala of a subject's nose. The nasal septum is oxygenated via a major arterial pathway from the heart, and hence is well-perfused even in the case of a patient with centralized perfusion. In particular, perfusion of the nasal septum is partly supplied by the ethmoidal arteries, which are branches from the internal carotid artery which is fed directly from the main aortic artery from the heart. The alar wing is supplied by branches from both the internal and external carotid arteries. The skin of the external nose receives arterial supply from branches of the maxillary and ophthalmic arteries. The septum and alar cartilages receive additional supply from the angular artery and lateral nasal artery. These are both branches of the facial artery (derived from the external carotid artery).

The disclosed nasal sensor may employ relatively short-wavelength light, e.g. 780 nm or shorter in wavelength in some embodiments and operates in transmission mode. It is recognized herein that due to the extreme thinness of the fleshy external portion of the nasal septum running from the tip of the nose to the lip to separate the nostrils, light at wavelengths of 780 nm or shorter has sufficient transmission through the nasal septum to provide PPG measurements with high intensity and good signal-to-noise ratio (SNR). Similar benefits can also be achieved by performing PPG measurements in transmission mode across the alar wing of a nostril.

In the case of a single-wavelength (or single light source) PPG device, suitable for measuring heart rate, in some preferred embodiments green light is used, in the range 590 nm or lower, and more preferably 530-580 nm. In order to perform pulse oximetry, PPG signals at two different wavelengths, with some significant wavelength separation, are used in deriving the arterial blood oxygen saturation value. In devices, systems and methods disclosed herein, both wavelengths may be at wavelengths of 780 nm or shorter. In some preferred embodiments, the two wavelengths are green light (590 nm or lower, and more preferably 530-580 nm) and red (or near-infrared) light (620-780 nm, and more preferably 640-740 nm). In other devices, systems and methods disclosed herein, one wavelength may be at a wavelength below 780 nm and the other wavelength may be at a wavelength equal to or greater than 780 nm.

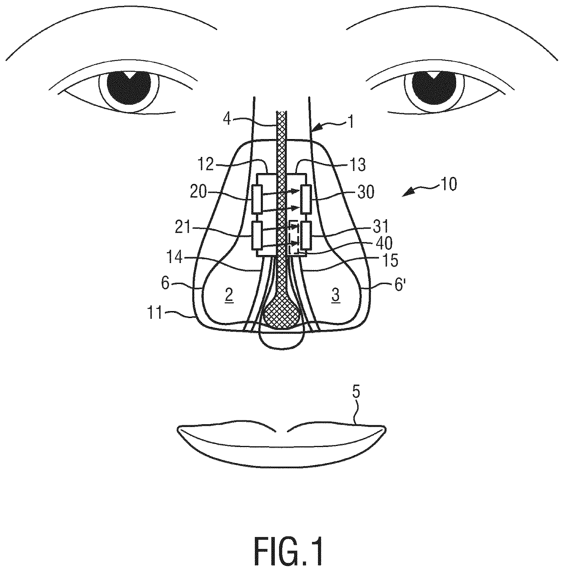

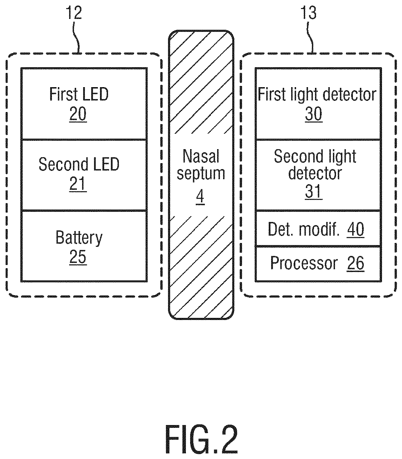

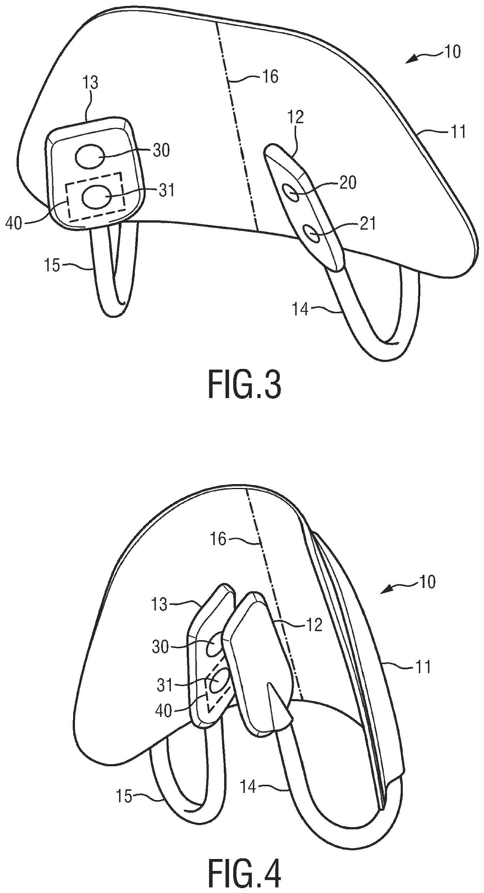

With reference to Figs. 1-4, an illustrative nasal sensor 10 according to the present invention is diagrammatically shown mounted on a nose 1 (Fig. 1), in block diagram format (Fig. 2), and in isolation perspective views shown open for mounting (Fig. 3) and closed (the state when secured to the nose, Fig. 4). The nasal sensor 10 may generally operate and provide physiological information like a pulse oximeter, but in addition provides information on respiratory flow direction.

With particular reference to Fig. 1, the nose 1 is a human nose which, in accordance with normal human anatomy, includes first and second nostrils 2, 3 separated by a nasal septum 4 that runs from the tip of the nose 1 to the lip 5 and separates the nostrils 2, 3. The lower outer parts of the nose are the alar wings 6, 6'.

The nasal sensor 10 is configured to be secured to the nose 1, and includes a nasal mount comprising an exterior frame 11 that when secured is located on the exterior of the nose 1, and first and second optical supports 12, 13 which when secured are inserted into respective first and second nostrils 2, 3. These supports 12, 13 may be mounted on respective extensions 14, 15 that are secured to the exterior frame 11 and, when secured to the nose 1, extend from the exterior frame 11 into the nostrils 2, 3 of the human nose 1. With the nasal sensor 10 secured to the nose 1, the nasal septum 4 of the nose 1 is interposed between the first optical support 12 and the second optical support 13. Fig. 1 diagrammatically illustrates components 11-15 of the illustrative nasal mount. Figs. 3 and 4 show isolation perspective views of the nasal mount including the exterior frame 11 and the first and second optical supports 12, 13 mounted on the respective extensions 14, 15.

In this embodiment the nasal sensor 10 is designed as a mechanical clip, in which the first optical support 12 and the second optical support 13 are first and second ends of the clip and are configured to clip onto the nasal septum 4 of the human nose 1 to secure the nasal sensor to the nose 1. The exterior frame 11 may include a spring-biased hinge 16 (e.g., with a bi-stable leaf spring or the like, not shown) which can be opened (Fig. 3) to move the two supports 12, 13 away from each other in order to unclip the device, and can be closed (Fig. 4) to bring the two supports 12, 13 together into sufficiently close proximity to each other to clip and hold the nasal septum 4 between the two supports 12, 13. In this clipped position (shown in Figs. 1 and 4) the PPG device is secured to the human nose 1 with the first optical support 12 and the second optical support 13 contacting opposite sides of the nasal septum 4.

In another contemplated mechanical mounting arrangement, the exterior frame 11 in its relaxed (unbiased) position has the two optical supports 12, 13 in close proximity to each other (as in Fig. 4), and the exterior frame 11 is springy, that is, has the ability to be manually opened to the open position of Fig. 3 by pressing the supports 12, 13 away from each other in order to position the supports over the nasal septum 4, after which the force is removed and the springy frame acts to return to the shape of Fig. 4 so as to clip the supports 12, 13 onto the nasal septum 4. In this embodiment there is no bi-stable hinge 16.

The illustrated nasal sensor mounting hardware is an example, and that more generally the nasal sensor may employ any mounting structure that is capable of being secured to a human nose with the nasal septum of the human nose interposed between a first optical support and a second optical support. In other contemplated embodiments, the PPG device mounting hardware is capable of being secured to a human nose with the alar wing 6 or 6' of a nostril of the human nose interposed between a first optical support and a second optical support. In such embodiments one optical support is suitably located inside the nostril while the other is outside the nostril, with the alar wing interposed between the optical supports inside and outside the nostril, respectively. In some embodiments transmission measurements are performed on both alar wings using two sets of optical supports, one for the alar wing of the left nostril and the other for the alar wing of the right nostril, and the transmission PPG measurements of the two alar wings and/ or the results derived from the PPG measurements are suitably averaged.

The nasal sensor 10 further includes a light emitter 20, 21 secured to the first support 12 arranged within the first nostril 2 and configured to emit light in at least one wavelength range towards the second nostril 3. In the embodiment shown in Figs. 1-4 the light emitter comprises two light sources 20, 21, e.g. two LEDs, emitting in different wavelength ranges. For instance, the first light source 20 may be configured to emit green light and the second light source 21 may be configured to emit red and/or infrared light. In another embodiment, the first light source 20 may be configured to emit red light and the second light source 21 may be configured to emit infrared light. Other wavelength ranges are possible as well. In another embodiment, a single light source may be used as light emitter, which is configured to emit light in a broad wavelength range (e.g. in the visible range, optionally with infrared light in addition) or in one or more narrow wavelength ranges (e.g. the wavelength ranges mentioned above).

Further, the nasal sensor 10 includes a light detector 30, 31 secured to the second support 13 and configured to detect light in the at least one wavelength range. In the embodiment shown in Figs. 1-4 the light detector comprises two light detection elements 30, 31, e.g. two photodiodes, sensing light in different wavelength ranges, in particular the wavelength ranges that are emitted by the light emitter(s) 20, 21. For instance, the first light detection element 30 may be configured to detect light in the wavelength range that is emitted by the first light source 20 and the second light detection element 31 may be configured to detect light in the wavelength range that is emitted by the second light source 21. The second light detection element 31 may generally be configured to detect light in the same wavelength range and/or a different wavelength range as the first light detection element 30. Further wavelengths, light sources and light detectors may be used as well. Alternatively, a single light detection element may be used on support 13, while using two or more light emitters on support 12, when control of the light emitters is multiplexed in time.

The nasal sensor 10 can thus measure both pulse (rate) and arterial blood oxygen saturation (SpO2). To measure the latter, in addition to a green light, red and/or near-infrared light is detected and evaluated. The red and infrared light (also called PPG components of corresponding PPG signals) can be used to compute the SpO2 value, while the green light (PPG component) can be used to measure a higher quality cardiac pulsatile component. In cases of severe centralization, the SpO2 measurement may drop out due to poor red and infrared signals, while a strong green pulsatile component can still be used to derive the cardiac pulse rate, or to do waveform analysis to infer estimates of the blood pressure and/or vascular tone of the patient.

With particular reference to Fig. 2, additional components of the nasal sensor 10 include a power source or connection to a power source and an electronic data processing device or operative connection with an electronic data processing device. In the embodiment shown in Fig. 2, the nasal sensor 10 is self-contained, and to this end includes an on-board battery 25 and an on-board electronic data processor 26, where the battery 25 powers the light sources 20, 21 and the electronic data processor 26 (e.g. a microprocessor, microcontroller, or other integrated circuit component). Such a self-contained nasal sensor 10 may output the SpO2 and pulse (rate) values via a data cable or wireless transmitter (not shown), and/or may include an on-board display (not shown, e.g. a low-power LCD display).

In some embodiments, the on-board battery 25 is omitted and the nasal sensor 10 is powered by a connected power cable. Similarly, in some embodiments the on-board electronic data processor 26 is omitted and the outputs of the light detectors 30, 31 and the control signals of the light sources 20, 21 are communicated via a cable (or in a wireless manner, e.g. via Bluetooth) to/from an off-board processor, such as a multi-function patient monitor. If communication with the off-board processor is wireless, the control signals of the light sources 20, 21 are communicated to a receiver and amplifier/current source connected to the light sources 20, 21 via a wire. As previously mentioned, if it is desired to measure only cardiac pulse (rate), but not oxygen saturation (SpO2), a single light source 20 and a single light detection element 30 may be sufficient.

The processor suitably derives one or more photoplethysmography, PPG, signal(s) from the light detected by the light detection element(s) and determines one or more vital signs from the PPG signal(s). Further, it determines the direction of respiratory flow through the subject's nose, and optionally respiration rate in addition, from the temporal variation of the amount of light detected by light detector, which may be the same light detection element (e.g. light detection element 30) based on which the PPG signals are derived or which may be a separate light detection element (e.g. the light detection element 31). For instance, pulse (rate), (optionally) SpO2 and (also optionally) respiration rate may be determined as follows from the PPG signals. The cardiac pulse rate is readily determined by measuring the peak-to-peak time interval of the pulsatile component of either a green light transmission or the red or near-infrared light transmission. In general, the measurement by green light is expected to be more accurate because green light is more strongly absorbed by arterial blood in the very thin nasal septum 4. If two or more wavelength channels are available, the values can be averaged together to increase accuracy and/or may be used as validity cross-checks.

If red and infrared light may be used, SpO2 can be detected from the detected light in these two wavelength ranges using the well-known ratio-of-ratios method. Respiration rate can be derived from the PPG signals by analyzing the variation in beat-to-beat intervals (respiratory sinus arrhythmia), analyzing the variation in the amplitudes of the cardiac pulses and by analyzing the baseline of the PPG signals. A more robust measurement of respiration rate can be obtained by combining the respiration rate derived from PPG signals with the respiration rate derived from the directional respiratory flows.

According to the present invention, the nasal sensor 10 further comprises one or more detection modification elements 40 secured to the second support 13 and configured to change the amount of light detected by the light detector based on the direction of flow of air within the second nostril 3. The one or more detection modification elements 40 can be implemented in various ways (including different configurations and/or locations) and are, hence, shown schematically only in Figs. 1-4. Various embodiments of the one or more detection modification elements 40 will be explained in the following. This changing amount of light is then used to detect the direction of respiratory flow, i.e. if the subject just inhales or exhales.

One main principle of the present invention is thus the use of a single nasal sensor mounted to an alar wing and/or the nasal septum by which different parameters can be measured from a single site:
- pulse rate (PR) and/or SpO2 and/or respiration rate (and, optionally, of other vital signs) from PPG signal(s) (preferably in the red and infrared wavelength range);
- direction-sensitive respiratory flow (e.g., useful in combination with an oxygen concentrator or CPAP to determine when to deliver O₂); and
- robust respiration rate (RR) derived from the direction-sensitive respiratory flow, wherein the direction allows for robustness to, e.g., only detect expiratory flows and ignore inspiratory flows.

Such a single-site measurement is beneficial for many patient groups, e.g., for COPD (chronic obstructive pulmonary disease) patients with an oxygen concentrator (to measure when to supply O₂), for distinction between central and obstructive sleep apnea, for ICU patients for early deterioration detection from RR, etc. RR derived from respiratory flow may also be fused with RR derived from PPG signals to make RR measurements more robust. Combining PPG/SpO2 and directional airflow/RR into a single sensor further has the advantage that it simplifies the workflow and improves patient comfort.

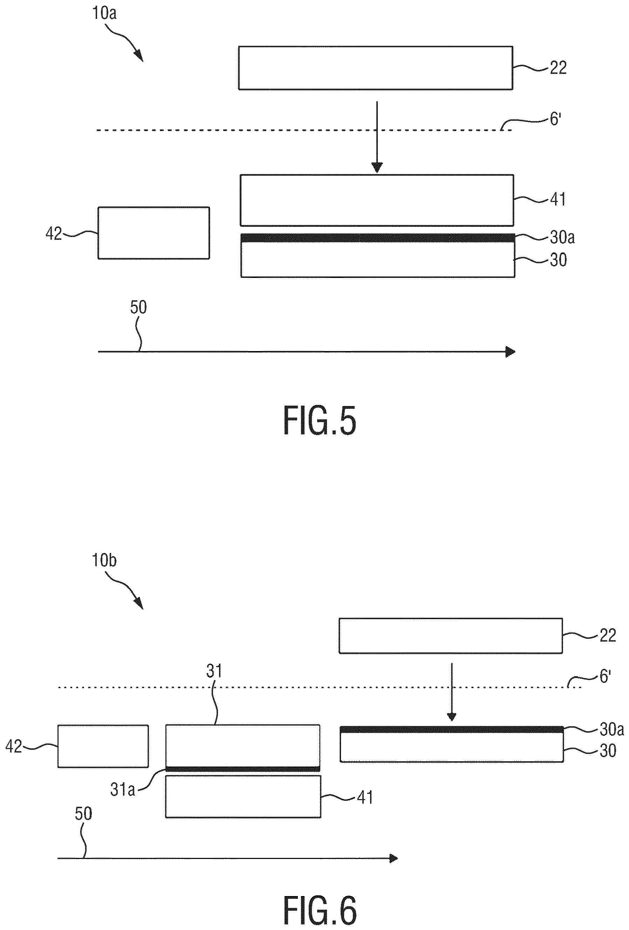

With reference to Figs. 5 to 10 various embodiments of the nasal sensor 10 according to the present invention are described. Only some main elements of these embodiments are shown schematically in these figures.

Fig. 5 shows a schematic diagram of a first embodiment of a nasal sensor 10a according to the present invention. In this embodiment a light emitter 22 (e.g. two LEDs representing the two light sources 20, 21) and a single light detection element 30 is provided. The light emitter 22 is arranged outside the nose at the outer side of the alar wing 6' (or 6) and the light detection element 30 is arranged inside the nostril 3 (or nostril 2) at the inner side of the alar wing 6' (or 6). In another embodiment the nasal sensor 10a may be arranged such that the light emitter 22 and the light detection element 30 are arranged on opposite sides of the nasal septum 4.

In this embodiment the one or more detection modification elements (40 in Figs. 1-4) comprise a thermosensitive element 41, which is implemented in this implementation as a thermosensitive component or layer or coating that is arranged on a front side 30a of the light detection element 30 facing the light emitter 22. The thermosensitive element 41 is configured to change its transmissivity for light depending on its temperature. In this way, the amount of light detected by the light detection element 30 is changed when air flows within the second nostril 6' in a first direction (e.g. when the patient exhales through the nose) compared to when air flows within the second nostril 6' in a second direction substantially opposite to the first direction (e.g. when the patient inhales), because exhaled air is generally warmer than inhaled air.

This effect can be further enhanced by optionally providing a heating element 42 that is configured to heat air flowing into the direction of the thermosensitive element 41. The heating element 42 may either be arranged between the opening of the second nostril 6' and the thermosensitive element 41 so that it heats up the inhaled air, or it may be arranged, seen from the opening of the second nostril 6', behind the thermosensitive element 41 so that it heats up exhaled air. In both cases the airflow in one direction 50 (i.e., inhaled air or exhaled air) is thus made warmer compared to the airflow in the opposite direction. The transmissivity of the thermosensitive element 41 and, thus, the amount of light sensed by the light detection element 30, changes in time with the respiration rate. This effect may, e.g., be detected as a change in the baseline of the PPG signal(s) derived from the detected light. Based on the measurement of the amount of light by the light detection element 30 (e.g., based on the change in the baseline of the PPG signal(s)) the respiration rate and the direction of respiratory flow through the nose can thus be determined.

The optional heating element 42 may be implemented using the heat of the light emitter 22 (e.g. of LEDs of the light emitter) or by use of a dedicated resistor or other heating means. The optional heating element is preferably useful in case of shallow breathing and/or small temperature differences between exhaled and ambient (inhaled) air.

Fig. 6 shows a schematic diagram of a second embodiment of a nasal sensor 10b according to the present invention. The one or more detection modification elements comprise a thermosensitive element 41 and an optional heating element 42 as well. But different from the nasal sensor 10a, the light detector comprises two light detection elements 30, 31. The first light detection element 30 is configured to detect light in the at least one wavelength range and the second light detection element 31 is configured to detect light in the same and/or a different wavelength range. The thermosensitive element 41 is arranged within or on or adjacent to the second light detection element 31, in particular on a light sensitive surface side 31a of the second light detection element 31. The light detected by the first light detection element 30 is thus used for deriving one or more PPG signals(s), from which one or more vital signs (in particular PR and SpO2) are determined. The light detected by the second light detection element 31 is used for determining the respiratory information, in particular respiration rate and respiratory flow direction in the way as explained above with respect to Fig. 5.

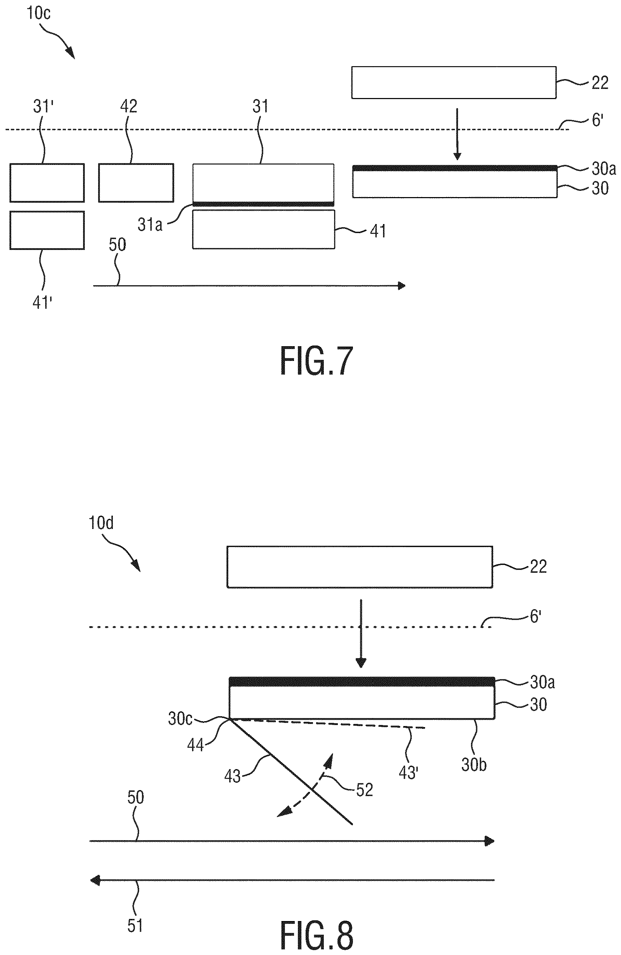

An alternative embodiment to the one shown in Fig. 6 is shown in Fig. 7 showing a nasal sensor 10c that includes two detection elements 31, 31', each with a thermosensitive element 41, 41', located on either side (in the direction of airflow) of the heating element 42. In still another embodiment (not shown) the two detection elements 31, 31' may instead be arranged on either side (in the direction of airflow) of the light emitter 22 (acting as heating source). These embodiments are more robust to detect the direction of the airflow and derive the respiration rate, as each of the two detection elements 31, 31' with thermosensitive element 41, 41' are most sensitive to one out of two directions of airflow. Specifically, in these embodiments the detection element with thermosensitive element is most sensitive to the direction of flow which first reaches the heating element 42 (or 22) and then the detection element with thermosensitive layer. Thereby, one of the two detection elements with thermosensitive layer can be used to detect expiratory flow and the other of the two detection elements with thermosensitive layer can be used to detect inspiratory flow.

In the three embodiments of the nasal sensor 10a, 10b, 10c, a contrast-enhancement element (not shown) may additionally be provided as part of or on top or close to the thermosensitive element 41, 41', which is configured to enhance the thermal contrast of the thermosensitive element 41, 41'. Such a contrast-enhancement element may, e.g., be a chemical layer, such as a hydrophilic layer. The idea of such a contrast-enhancement element is to be sensitive, e.g., to humidity adding a cooling effect because of vaporization heat and to enhance the temperature change. Other options are, e.g., to be sensitive to the CO₂ levels, where the CO₂ adsorbs/desorbs and provides heating effects.

Fig. 8 shows a schematic diagram of a fourth embodiment of a nasal sensor 10d according to the present invention. In this embodiment the light detector comprises a single light detection element 30. The one or more detection modification elements comprise an opto-mechanical unit, in this case in the form of a lid 43 (or a shutter) that is arranged on a rear side 30b of the light detection element 30 facing away from the light emitter 22. The lid 43 is configured to move - as indicated by the arrow 52 - between a first position (as shown in Fig. 8 by the dashed line 43a), in which the lid 43 is arranged on or adjacent to the rear side 30b of the light detection element 30 (for instance, substantially in parallel to the rear side 30b), and a second position (as shown in Fig. 8 by the solid line 43), in which the lid 43 is arranged at a distance from or inclined to the rear side 30b of the light detection element 30.

The lid 43 is thus configured to change its position and/or orientation with respect to the light detection element 30 depending on the direction of the flow of air within the second nostril 3. In particular, the lid 43 can move between the first position (dashed line 43a) and/or orientation with respect to the light detection element 30, when the air flows within the second nostril 3 in a first direction 50, and a second position (solid line 43) and/or orientation with respect to the light detection element 30, when the air flows within the second nostril 3 in a second direction 51 substantially opposite to the first direction 50.

This movement of the lid 43 is achieved in an embodiment, where the lid 43 is hinged or mounted with one end 44 to the light detection element 30, in particular to an edge 30c of the light detection element 30, and is configured to rotate within an angular range around its end 44 in response to the flow of air within the second nostril 3.

The surface of the lid 43 facing the light detection element 30 is preferably covered with or made of light-reflective material, e.g. a layer made of silver or other metallic material, or the lid 43 is completely made of light-reflective material. Hence, in the first position (43a) more light will be reflected back onto the light detection element 30 and in the second position (43) less light will be reflected back onto the light detection element 30. Based on the additional amount of light reflected onto the light detection element 30, the direction of airflow can be determined.

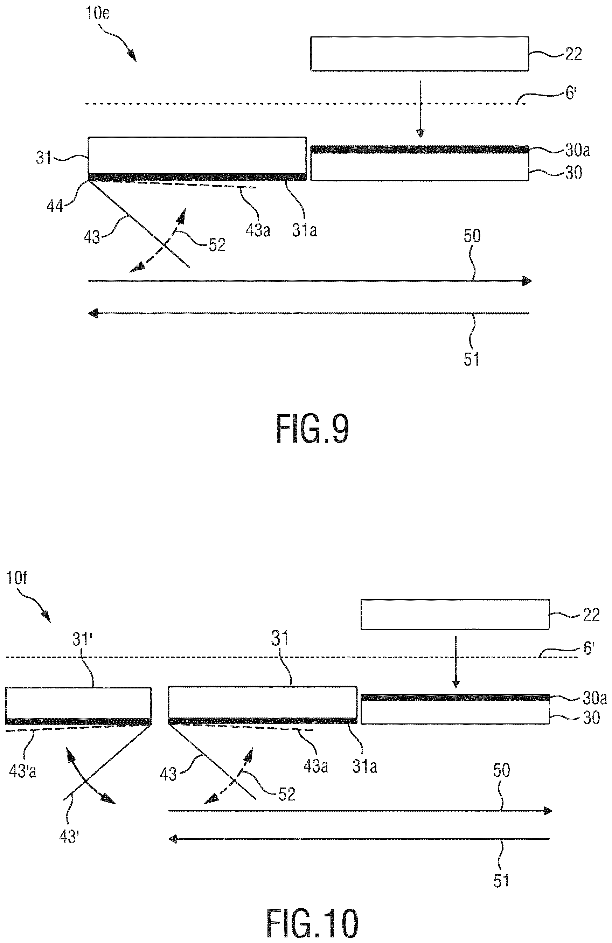

Fig. 9 shows a schematic diagram of a fifth embodiment of a nasal sensor 10e according to the present invention. In this embodiment the light detector comprises two light detection elements 30, 31, wherein the first light detection element 30 is configured to detect light in the at least one wavelength range and the second light detection element 31 is configured to detect light in the same and/or a different wavelength range. The lid 43 is arranged on a light sensitive surface side 31a of the second light detection element 31. As explained with respect to Fig. 8, the lid 43 is configured to move between a first position (dashed line 43a), in which the lid is arranged on or adjacent to the light sensitive surface side of the second light detection element 31, and a second position (solid line 43), in which the lid 43 is arranged at a distance from or inclined to the light sensitive surface side 31a of the second light detection element 31.

An alternative embodiment to the one shown in Fig. 9 is shown in Fig. 10 showing a nasal sensor 10f that includes two detection elements 31, 31', optionally arranged next to each other, each with an opto-mechanical unit 43, 43', where the opto-mechanical units are arranged on opposing sides of the light sensitive surfaces of the two detection elements 31, 31'. By arranging the opto-mechanical units 43, 43' on opposing sides of the two detection elements 31, 31', the units behave oppositely for each direction of airflow, so the direction of airflow can be explicitly measured from both detection elements 31, 31' and the onsets of inhalation and exhalation can be explicitly determined, which allows for more robust determination of respiration rate. This is specifically of interest when there is a pause in respiration, i.e., when respiratory flow ceases for a portion of the respiratory cycle, during which period of time the lid 43 can already move to the second position due to gravitational pull instead of due to airflow. Having lid 43 in a second position may in that situation no longer be only indicative of a specific airflow. By having two detection elements 31, 31' with an opto-mechanical unit 41, 41' where the opto-mechanical unit 43 moves to a first position during inhalation and the other opto-mechanical unit 43' moves to a first position during exhalation, the onsets of inhalation and exhalation can be explicitly measured, allowing for more robust assessment of respiration rate.

Apart from the different construction and function of the detection modification elements, the function and operation of the light detection and the determination of vital signs and respiratory information are substantially the same as explained above with respect to the embodiments shown in Figs. 5 to 10.

As the presented solutions using a direction-sensitive flow measurement allow for a more detailed analysis of the outflowing and inflowing air, the respiratory pattern can be analyzed in more detail. Changes in aspects of the respiratory pattern over time, such as the inhalation-exhalation ratio, can thus be detected. Further, the collection of data over time allows to establish an individual respiration pattern specific for the monitored subject, which in turn can be used to detect possible changes and/or deviations in the respiratory pattern over time. In case such changes or deviations in the respiration pattern are detected, a clinician or caregiver can be informed or alerted about this.

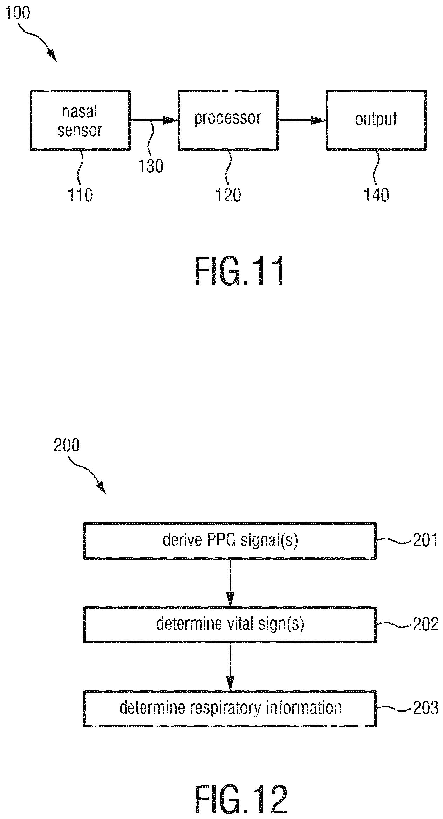

Fig. 11 shows a schematic diagram of an embodiment of a system 100 for determining physiological information of a subject according to the present invention. The system comprises a nasal sensor 110, which may be configured as disclosed herein and explained above with reference to Figs. 1-10, but which may also be a nasal sensor without the one or more detection modification elements. Thus, the nasal sensor 110 comprises at least a nasal mount configured to be secured to nose of a subject and including a first support and a second support with the nasal septum or an alar wing interposed between the first support and the second support and with the first support arranged within a first nostril or outside the nostrils at an alar wing of the second nostril and with the second support arranged inside the second nostril. Further, the nasal sensor 110 comprises a light emitter secured to the first support and configured to emit light in at least one wavelength range towards the second nostril and a light detector secured to the second support and configured to detect light in the at least one wavelength range. The system 100 further comprises a processing unit 120 configured to derive a PPG signal (or multiple PPG signals) from the light detected by the light detector, to determine one or more vital signs from the PPG signal and to determine the direction of respiratory flow through the subject's nose (and optionally respiration rate), from the temporal variation of the amount of light detected by the light detector.

Still further, the system 100 comprises a signal line 130, e.g. a cable or wireless transmission, configured to provide a detection signal representing the light detected by the light detector to the processing unit 120 and to provide the control signals from the processing unit 120 to the light sources. If communication with processing unit 120 the off-board processor is wireless, the control signals of the light sources 20, 21 are communicated to a receiver and amplifier/current source connected to the light sources 20, 21 via a wire.

Optionally, an output unit 140 may be provided for outputting the determined information. The output unit may, e.g., be a display for displaying the determined information, optionally with the ability to provide audible alarms, or an interface or transmission unit for providing the determined information to another device, e.g. an oxygen concentrator or CPAP device, for use in the control of the other device.

Fig. 12 shows a flowchart of an embodiment of a method 200 for determining physiological information of a subject according to the present invention. As explained above with respect to the system 100, the method uses a nasal sensor 110 which may be configured as disclosed herein and explained above with reference to Figs. 1-10, but which may also be a nasal sensor without the one or more detection modification elements. In a first step 201, a PPG signal (or multiple PPG signals) is derived from the light detected by the light detector. In a second step 202, one or more vital signs are determined from the PPG signal(s). In a third step 203, which may be carried out in parallel to (or before or after) step 202, the direction of respiratory flow through the subject's nose (and optionally respiration rate) is determined from the temporal variation of the amount of light detected by the light detector.

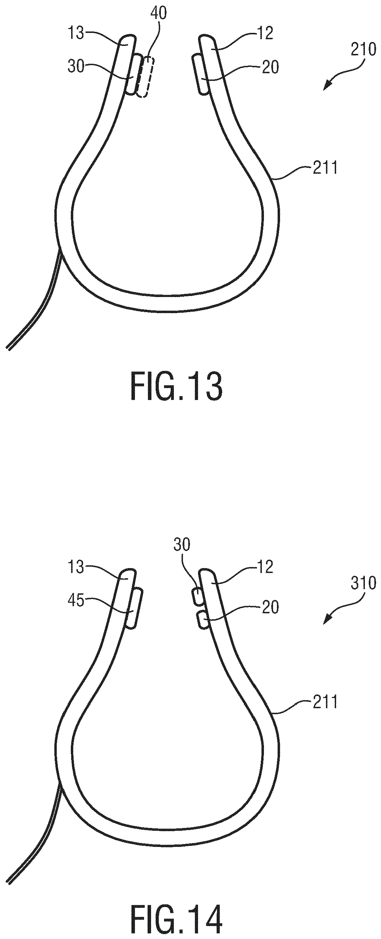

Fig. 13 shows a schematic diagram of a seventh embodiment of a nasal sensor 210 according to the present invention. According to this embodiment, the nasal sensor 210 is configured to be applied to an alar wing rather than to the nasal septum as the embodiment shown in Figs. 1-4. The nasal mount 211 is shaped as part of a ring with extensions forming a first support 12 carrying the light emitter 20 and being arranged at the outer surface of the nasal wing (i.e., outside the nostril) and a second support 13 carrying the light detector 30 and the one or more detection modification elements 40 and being arranged at the inner surface of the nasal wing (i.e., within the nostril). The one or more detection modification elements 40 are shown schematically in Fig. 13 and may be configured and arranged as explained above.

Fig. 14 shows a schematic diagram of an eighth embodiment of a nasal sensor 310 according to the present invention. According to this embodiment, the nasal sensor 310 is configured to be applied to an alar wing and may be shaped as part of a wing with extensions like the embodiment shown in Fig. 13. The first support 12 is arranged at the outer surface of the nasal wing (i.e., outside the nostril) and carries both the light emitter 20 and the light detector 30. The second support 13 is arranged at the inner surface of the nasal wing (i.e., within the nostril) and carries the one or more detection modification elements 45.

In this embodiment the one or more detection modification elements 45 are configured to reflect light emitted through the nasal wing by the light emitter 20 back through the nasal wing to the light detector 30. Furthermore, based on the temperature of the air flowing through the nostril, in which the second support 13 is arranged, the reflectivity of the reflective element 45 changes. Thus, the amount of light detected by the light detector 30 changes due to the changes of the reflectivity of the reflective element 45, which hence allows detection if the subject inhales or exhales.

The one or more reflective detection modification elements 45 may be configured as reflective pad, reflective layer or reflective foil, in general any material that changes its reflectivity based on its temperature, i.e., where the reflectivity of the material on the inside of the nose gets modulated by temperature of inhaled and exhaled air. For instance, liquid crystal thermofoils, or material that changes the polarization of light based on temperature, or a material as used in "self-coloring corrective glasses" may be used.

In summary, the present invention makes use of a nasal sensor with direction-sensitive respiratory-airflow detection. A flow measurement sensitive to flow direction allows distinguishing between outflowing and inflowing air. This enables robust detection of only exhalations and allows for determination of respiration rate which is robust to disturbing external (in-going) airflows. At the same time, one or more vital signs of the subject can be determined by use of the same nasal sensor.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Nasal sensor (10, 110, 210) comprising:
- a nasal mount (11, 12, 13) configured to be secured to nose of a subject and including a first support (12) and a second support (13) with the nasal septum (4) or an alar wing (6, 6') interposed between the first support and the second support and with the first support (12) configured to be arranged within a first nostril or outside the nostrils at an alar wing of the second nostril and with the second support (13) configured to be arranged inside the second nostril;
- a light emitter (20, 21) secured to the first support and configured to emit light in at least one wavelength range towards the second nostril;
- a light detector (30, 31) secured to the first or second support and configured to detect light in the at least one wavelength range; and
- one or more detection modification elements (40, 41, 42, 43, 45) secured to the second support and configured to change the amount of light detected by the light detector based on the direction of flow of air within the second nostril.

2. Nasal sensor according to claim 1,
wherein the one or more detection modification elements (40, 41, 42, 43, 45) are configured and/or located to change the amount of light detected by the light detector when air flows within the second nostril in a first direction compared to when air flows within the second nostril in a second direction substantially opposite to the first direction.

3. Nasal sensor according to any one of the preceding claims,
wherein the one or more detection modification elements comprise a thermosensitive element (41), in particular a thermosensitive component or layer or coating, configured to change its transmissivity for light depending on its temperature.

4. Nasal sensor according to claim 3,
wherein the light detector comprises a single light detection element (30) and
wherein the thermosensitive element (41) is arranged on a front side of the light detection element facing the light emitter.

5. Nasal sensor according to claim 3,
wherein the light detector comprises two light detection elements (30, 31), wherein a first light detection element is configured to detect light in the at least one wavelength range and a second light detection element is configured to detect light in the same and/or a different wavelength range, and
wherein the thermosensitive element (41) is arranged within or on or adjacent to the second light detection element, in particular on a light sensitive surface side of the second light detection element

6. Nasal sensor according to any one of claims 3 to 5,
wherein the one or more detection modification elements further comprise a heating element (42) configured to heat air flowing into the direction of the thermosensitive element, in particular a heating element arranged between the opening of the second nostril and the thermosensitive element or, seen from the opening of the second nostril, behind the thermosensitive element, and/or a contrast-enhancement element configured to enhance the thermal contrast of the thermosensitive element.

7. Nasal sensor according to any one of the preceding claims,
wherein the one or more detection modification elements comprise a lid (43) configured to change its position and/or orientation with respect to the light detector depending on the direction of flow of air within the second nostril.

8. Nasal sensor according to claim 7,
wherein the lid (43) is configured to move between a first position and/or orientation with respect to the light detector, when the air flows within the second nostril in a first direction, and a second position and/or orientation with respect to the light detector, when the air flows within the second nostril in a second direction substantially opposite to the first direction.

9. Nasal sensor according to claim 7 or 8,
wherein the light detector comprises a single light detection element (30),
wherein the lid (43) is arranged on a rear side of the light detection element facing away from the light emitter, and
wherein the lid (43) is configured to move between a first position, in which the lid is arranged on or adjacent to the rear side of the light detection element, and a second position, in which the lid is arranged at a distance from or inclined to the rear side of the light detection element.

10. Nasal sensor according to claim 7 or 8,
wherein the light detector comprises two light detection elements (30, 31), wherein a first light detection element is configured to detect light in the at least one wavelength range and a second light detection element is configured to detect light in the same and/or a different wavelength range, and
wherein the lid (43) is arranged on a light sensitive surface side of the second light detection element, and
wherein the lid (43) is configured to move between a first position, in which the lid is arranged on or adjacent to the light sensitive surface side of the second light detection element, and a second position, in which the lid is arranged at a distance from or inclined to the light sensitive surface side of the second light detection element.

11. Nasal sensor according to claim 9 or 10,
wherein the lid (43) is hinged or mounted with one end (44) to the light detection element or the second light detection element, respectively, in particular to an edge of the respective light detection element, and is configured to rotate within an angular range around its end in response to the flow of air within the second nostril.

12. Nasal sensor according to any one of the preceding claims,
wherein the light emitter (20, 21) and the light detector (30, 31) are secured to the first support configured to be arranged outside the nostrils at an alar wing of the second nostril, and
wherein the one or more detection modification elements (45) are configured to reflect light emitted by the light emitter to the light detector and to change the reflectivity based on the direction of flow of air within the second nostril.

13. Nasal sensor according to any one of the preceding claims, wherein
the light emitter (20, 21) is configured to emit light in at least two or more different wavelength ranges towards the second nostril and the light detector (30, 31) is configured to detect light in the at least two wavelength ranges; and/or
wherein the nasal sensor further comprises a processing unit (26) secured to the first support or the second support and configured to derive a photoplethysmography, PPG, signal from the light detected by the light detector and to determine a vital sign, in particular pulse rate and/or SpO2 and/or respiration rate, from the PPG signal and the direction of respiratory flow through the subject's nose, and optionally respiration rate, from the temporal variation of the amount of light detected by the light detector.

14. System (100) for determining physiological information of a subject, the system comprising:
- a nasal sensor (110), in particular according to any one of the preceding claims, the nasal sensor comprising:
a nasal mount configured to be secured to nose of a subject and including a first support and a second support with the nasal septum or an alar wing interposed between the first support and the second support and with the first support arranged within a first nostril or outside the nostrils at an alar wing of the second nostril and with the second support arranged inside the second nostril;
a light emitter secured to the first support and configured to emit light in at least one wavelength range towards the second nostril; and
a light detector secured to the first or second support and configured to detect light in the at least one wavelength range;
- a processing unit (120) configured to derive a photoplethysmography, PPG, signal from the light detected by the light detector, to determine one or more vital signs from the PPG signal and to determine the direction of respiratory flow through the subject's nose, and optionally respiration rate, from the temporal variation of the amount of light detected by the light detector; and
- a signal line (130) configured to provide a detection signal representing the light detected by the light detector to the processing unit.

15. Method for determining physiological information of a subject by use of a nasal sensor, in particular according to any of claims 1 to 13, the nasal sensor comprising:
a nasal mount configured to be secured to nose of a subject and including a first support and a second support with the nasal septum or an alar wing interposed between the first support and the second support and with the first support arranged within a first nostril or outside the nostrils at an alar wing of the second nostril and with the second support arranged inside the second nostril;
a light emitter secured to the first support and configured to emit light in at least one wavelength range towards the second nostril; and
a light detector secured to the first or second support and configured to detect light in the at least one wavelength range,
the method comprising:
- deriving a photoplethysmography, PPG, signal from the light detected by the light detector;
- determining one or more vital signs from the PPG signal; and
- determining the direction of respiratory flow through the subject's nose, and optionally respiration rate, from the temporal variation of the amount of light detected by the light detector.
